# EUROPEAN PATENT APPLICATION

(11) **EP 4 343 777 A1**
(43) Date of publication of application: **27.03.2024**
(21) Application number: 22197011.4
(22) Date of filing: 22.09.2022
(51) Int. Cl.: G16H 20/40, G16H 30/40, G16H 50/50, G16H 50/70, G06N 3/045, A61B 34/20, A61B 17/56

(54) **TECHNIQUE FOR SELECTING A MACHINE-TRAINED MODEL FOR DETERMINING AN IMPLANT-RELATED PARAMETER**

(71) Applicant: Stryker European Operations Limited, Carrigtwohill, Co. Cork T45 HX08 (IE)
(72) Inventor: WEEDE, Oliver, 79110 Freiburg (DE); HERRMANN, Florian, 77963 Schwanau (DE)
(74) Representative: Röthinger, Rainer

(57) **Abstract**

A computer-implemented technique for a user-specific selection of a machine-trained model for determining an implant-related parameter is presented. A set of different machine-trained models is provided. Each model is indicative of a dedicated implant-related parameter for a dedicated patient anatomy. A method implementation of the technique comprises the steps of receiving first patient image data indicative of a patient anatomy in which an implant is to be placed and applying at least one first model from the set of models on the first patient image data to determine an implant-related parameter. The method also includes suggesting the determined implant-related parameter to a dedicated user and receiving, from the user, feedback on the suggested implant-related parameter. The user feedback comprises one of a confirmation of the suggested implant-related parameter and an adaptation thereof. Further still, the method includes selecting, based on the user feedback, at least one second model from the set of models that is to be applied on second patient image data.

## Description

### Technical Field

The present disclosure generally relates to the field of computer-assisted surgery. In particular, a computer-implemented technique for a user-specific selection of a machine-trained model for determining an implant-related parameter Is presented. Further, a computer-implemented technique for machine-training a set of different models is presented, wherein each model is configured for determining a implant-related parameter with respect to a patient anatomy. The techniques presented herein can be practiced in the form of methods, computer program products and apparatuses.

### Background

Many surgical procedures rely on the placement of an implant in bone. In spine surgery, for example, fixation screws (e.g., pedicle screws, sacral screws or cortical screws) are implanted into vertebrae to hold a rod that stabilizes the spine. Proper selection and appropriate placement of the fixation screws is critical to the outcome of the surgery, as the fixation screws must provide adequate stability for ventral and dorsal aspects of the spine. Incorrect implant placement can seriously injure the patient. In the example of spine surgery, an improperly placed pedicle screw can lead to so-called breaching and other injuries. Similar issues arise in the context of other implants, such as bone plates or interbody cages.

Ideal implant placement depends on many factors, including the morphology of the bone to be treated, the specific anatomy of a particular patient, patient age and size, degenerative diseases, and so on. It has been found that ideal implant placement also depends on the treating surgeons, for example with respect to their preferences and skills. There exist solutions for automatically determining an implant position based on patient image data and presenting the position to the treating surgeon for confirmation (e.g., by superimposing a suggested implant position on patient image data). It has been found that the automatically determined implant position will be often manually revised and adjusted by the surgeon. In pedicle screw fixation, for example, the transverse angle (axis angle) may vary within a certain range depending on the surgeon's preference. There are also typical screw diameters and lengths chosen for different spinal levels, such as 6 x 50 mm or 6.5 x 50 mm for the lumbar spine and 5 x 45 mm or 5.5 x 45 mm for the thoracic spine. Some surgeons prefer the same diameter and length for the left and right pedicles, while others focus on choosing the ideal length and diameter for each side, resulting in an asymmetrical selection. Not only screw placement, but also screw selection is therefore surgeon-dependent.

Each manual revision and adjustment of automatically determined implant selections and positions is associated with a cognitive load on the treating surgeon and consumes mental resources and time. It would thus be desirable to avoid or at least reduce such manual interactions. Moreover, it would in some instances be desirable to assist (in particular less experienced) surgeons in terms of selecting and positioning an implant based on the surgical practices of other (in particular more experienced) surgeons. The overall goal is to reduce the health risks for the patient as well as the time and efforts required for a surgical intervention in which an implant is placed.

### Summary

There is a need for computer-implemented technique for determining an implant-related parameter that avoids one or more of the above or other drawbacks.

According to a first aspect, a computer-implemented method for a user-specific selection of a model for determining an implant-related parameter is provided. In this context, a set of different machine-trained models is provided. Each model is indicative of a dedicated implant-related parameter for a dedicated patient anatomy. The method comprises the steps of receiving first patient image data indicative of a patient anatomy in which an implant is to be placed, applying at least one first model from the set of models on the first patient image data to determine an implant-related parameter, and suggesting the determined implant-related parameter to a dedicated user. The method further comprises receiving, from the user, feedback on the suggested implant-related parameter, wherein the user feedback comprises one of a confirmation of the suggested implant-related parameter and an adaptation thereof. Further still, the method comprises selecting, based on the user feedback, at least one second model from the set of models that is to be applied on second patient image data.

The first patient image data may be processed prior to applying the at least one first model from the set of models thereon. As an example, the image data may be segmented or processed otherwise to determine first image information representative of the patient anatomy in which an implant is to be placed. The first image information may be indicative of a contour of the patient anatomy (e.g., a bone contour). To this end, an image analysis technique may be used. If, for example, the first patient image data are computer tomography (CT) data, the segmenting may be based on an analysis (e.g., thresholding) of the Hounsfield values comprised by the CT data. For example, the levels of vertebrae that are to be stabilized may be identified based on the first image information (and, optionally, additional user input). The model selection may then also be based on the vertebra levels. The segmentation or other processing of the image data, e.g., identifying vertebral levels and/or highlighting points within a patient anatomy, may be performed automatically, e.g., by utilizing one or more trained neural networks.

In one variant, the method may comprise selecting the at least one first model based on at least one first historical data item associated with the user and indicative of at least one third model of the set of models. The at least one first (and any further) historical data time may thus be user-specific. In some implementations, the first historical data item may be a data structure that comprises an entry for two or more models, or each model, of the set of models. The entries may be weighted, i.e., indicative of a relevance of the respective models (e.g., as defined by a weighting factor between 0 and 1). Further, the at least one first historical data item may be associated with a specific patient anatomy. In other words, different anatomies (e.g., different bones or bone groups) may be associated with different first historical data items (and, optionally, different models in the model set). For example, different vertebrae or different sets of vertebrae may be associated with different first historical data items.

Additionally or alternatively, the method may comprise generating at least one second historical data item associated with the user and indicative of the at least one second model. By generating new historical data items indicative of the user, the model selection step may be adapted according to the personal preferences and techniques of the user. A change of the personal preferences and techniques of the user over time may thus also be taken into account.

Further, there may exist multiple first historical data items associated with a dedicated user, e.g., up to 5 items, up to 10 items or more than 10 items. In this case, selecting the at least one first model may comprise at least one of determining the model most often indicated in the multiple first historical data items, and an interpolation based on at least some of the models indicated in the multiple first historical data items. The interpolation may be based on a number of times a model is indicated. Additionally or alternatively, the interpolation may be based on weighting factors (e.g., as explained above). The concrete implementation of the interpolation may depend on the nature of the machine-trained models. If, for example, the models take the form of neural networks, the outputs of two or more of the neural networks may be interpolated. In such or other variants, the interpolation may be based on a linear combination, optionally based on the weighting factors.

In one variant, the user feedback may comprise an adaptation of the suggested implant-related parameter. In this case, the step of generating the at least one second historical data item may comprise utilizing each of the models from the model set to determine, based on the first patient image data, a respective implant-related parameter. For example, each of the models may be applied on the first patient image data. The step of generating the at least one second historical data item may further comprise determining, for each respective implant-related parameter, a respective fit to the adapted implant-related parameter. The at least one second historical data item may then be generated based on one or more of the respective fits. For example, the at least one second historical data item may comprise weighted entries for two or more models of the set of models, with the weighting factors being determined based on the respective fits (e.g., a better fit leading to a higher weighting factor). In another example, the at least one second historical data item may be indicative of the model having the best fit.

In one variant, the first model may be randomly selected from the set of models. In another variant, the first model may be selected based on the preference of a specific group of users, e.g., the surgeons of a particular hospital or the surgeons of a specific division of the hospital. For example, the first model may be the model preferred by most of the surgeons of a hospital. In another example, the first model may be the model preferred by a selected group of users, e.g., specialists for a specific medical field or of a division of the hospital. The selection of the first model based on a randomly selected model or based on the preference of a specific group of users may be utilized in case no user-specific historical data item exists beforehand, e.g., when a user uses triggers the computer-implemented method for the first time, or when hospital management wants to implement specific guidelines for specific surgeries.

In one variant, the method may comprise receiving auxiliary information indicative of at least one of a patient-related parameter (e.g., patient diagnosis, patient age, size, weight or sex, etc.) and a user-related parameter (e.g., surgical division of the user, hospital associated with the user, etc.). In some implementations, a dedicated set of models is provided for different parameters or parameter ranges in terms of at least one of the patient-specific parameter and the user-specific parameter. In this case, the model selection may be also based on the received auxiliary information. The auxiliary information may improve patient-specific implant selection, e.g., since the bone density of a patient may differ based on age and diagnosis and may require a specific determination of the implant-related parameter.

The auxiliary information may be reflected in the set of models, i.e., the models may be machine-trained based at least in part on training data indicative of the auxiliary information. As a result, a different set of models may be provided to different surgical divisions or applied for different sexes.

Additionally or alternatively, each historical data item may be indicative of the auxiliary information. In such a case, the at least one first model may be selected based on the at least one first historical data item that is associated with the user and indicative of the at least one third model of the set of models and of the auxiliary information.

In one variant, the implant may be a bone screw (e.g., a pedicle screw) and the dedicated implant-related parameter may be indicative of at least one of a dedicated orientation of the screw relative to the patient's anatomy, a dedicated position of a head of the screw relative to the patient's anatomy, a dedicated position of a tip of the screw relative to the patient's anatomy, a dedicated screw length and a dedicated screw diameter. A screw orientation of screw head/position relative to the patient's anatomy may be indicated (e.g., output to the user) relative to the first patient image data or a processed (e.g., segmented) version thereof. In other variants, the implant may be a bone plate and the dedicated implant-related parameter may be indicative of at least one of dedicated plate orientation relative to the patient's anatomy, a dedicated plate length and a dedicated plate thickness. In still other variants, the implant may be an intervertebral disc implant, i.e., cage, and the dedicated implant-related parameter may be indicative of at least one of cage orientation relative to the patient's anatomy, e.g., a lordotic angle of the cage, the dimensions and the form of the cage. The model selection may be based on determining a dedicated implant-related parameter that optimizes at least one of a pelvic incidence, a pelvic tilt and a sacral slope. The first patient image data or other patient image data may comprise fluoroscopy images, i.e., fluoroshots.

In one variant, the patient anatomy may be a spine and there exists a dedicated set of models for each of multiple sets of one or more vertebrae of the spine that are to be treated. In this case, the model selection may further comprise receiving a user input on a set of one or more vertebrae to be treated, and the model selection may be also based on the user input. For example, each vertebrae set may be indicative of a specific sequence of vertebrae that are to be stabilized via a rod. The user input may be received relative to a visualization of the first patient image data (e.g., relative to a segmented view thereof) on a display device.

In one variant, suggesting the determined implant-related parameter to the user may comprise visualizing the implant-related parameter relative to the first patient image data. The method may comprise associating the implant-related parameter (e.g., an implant orientation) with a coordinate system of the first patient image data (i.e., relative to the patient's anatomy). The method may comprise generating display instructions based on the implant-related parameter. The display instructions may be configured to visualize at least one of the implant and the implant-related parameter. In some implementations, the display instructions are configured to visualize at least one of a dedicated orientation of the implant relative to the patient's anatomy (e.g., superimposed on the first patient image data).

The method may comprise processing the first image data based on the determined implant-related parameter. The display instructions may be indicative of the processed image data. In some implementations, processing of the image data comprises at least one of orienting the image data (e.g., dependent on a screw trajectory) and zooming into the image data (e.g., with an image center defined by the implant).

According to a second aspect, a computer-implemented method for machine-training a set of different models is provided. Each model is configured for determining a dedicated implant-related parameter with respect to a dedicated patient anatomy. The method comprises receiving a set of training data indicative of implant-related parameters determined by a variety of users relative to a respective patient anatomy.

The method also comprises dividing the training data into dedicated classes, wherein each class is indicative of a dedicated implant-related parameter or parameter range. The method further comprises machine-training a set of different models, wherein each model is trained based on the training data of a dedicated class.

The training data may be based on a image data acquired for multiple patients and include implant-related parameters. The implant-related parameters may be indicative of implants placed or confirmed by multiple specialists, e.g., multiple highly specialized and experienced users. Additionally or alternatively, the training data may be based on implant placements that have shown the best results in the aftercare of a patient.

In one variant, dividing the training data may be done responsive to a manual input. For example, a surgeon may define the classes based, e.g., on at least one of personal experience and predefined rules. Additionally or alternatively, dividing the training data is performed at least partially automatically based on a similarity metric for the implant-related parameter. Dividing the training data based on the similarity metric may comprise performing a cluster analysis of the training data. For example, vertebral levels may be mapped to a reference spine and a cluster analysis may be performed on screw positions (e.g., with a single linkage hierarchical clustering algorithm). The similarity metric may define a weighted sum of the differences between a head and a tip position and a screw diameter.

In one variant, the method according to the second aspect may further comprise receiving additional training data indicative of one or more implant-related parameters that have been adapted in accordance with the method according to the first aspect, and retraining one or more of the models based on the additional training data. To avoid that the retraining leads to a deterioration of the models, the additional training data may need to be confirmed before they used for the re-training.

A user may select between different data that may be used for retraining of the models. For example, the user may select between (i) delta parameters, (ii) anonymized metrics, (iii) reduced imaging data volume, and (iv) full Protected Health Information (PHI) image data. This selection may be based on privacy policies regarding the patient data. For example, in case i) only implant-related parameter data may be provided, e.g., screw head and tip adaptions (e.g., in form of a vector). In case ii) anonymized patient anatomy data may be provided, like the relative positions of a vertebra to a screw. In case iii) partial image data may be provided and in case iv) full anonymized patient data may be provided.

In one variant, the training data may be indicative of the implant-related parameters relative to a segmented contour of the patient anatomy. As explained above, the contour may be a bone contour (e.g., a contour of one or more vertebrae).

According to a third aspect, a computer program product is provided. The computer program product comprises program code portions configured to cause a processor to carry out a method of any of the preceding claims when the program code portions are executed on the processor. The computer program product may be stored on a computer-readable recording medium.

According to a fourth aspect, an apparatus for a user-specific selection of a model for determining an implant-related parameter is provided. Further, a set of different machine-trained models is provided. Each model is indicative of a dedicated implant-related parameter for a dedicated patient anatomy. The apparatus is configured to receive first patient image data indicative of a patient anatomy in which an implant is to be placed, to apply at least one first model from the set of models on the first patient image data to determine an implant-related parameter, and to suggest the determined implant-related parameter to a dedicated user. The apparatus is further configured to receive, from the user, feedback on the suggested implant-related parameter, wherein the user feedback comprises one of a confirmation of the suggested implant-related parameter and an adaptation thereof. Further still, the apparatus is configured to select, based on the user feedback, at least one second model from the set of models that is to be applied on second patient image data.

According to a fifth aspect an apparatus for machine-training a set of different models is provided. Each model is configured for determining a dedicated implant-related parameter with respect to a dedicated patient anatomy. The apparatus is configured to receive a set of training data indicative of implant-related parameters determined by a variety of users relative to a respective patient anatomy and to divide the training data into dedicated classes, wherein each class is indicative of a dedicated implant-related parameter or parameter range. Further, the apparatus is configured to machine-train a set of different models, wherein each model is trained based on the training data of a dedicated class.

The apparatuses according to the fourth and fifth aspects may be configured to perform any of the method steps according to the first and second aspects presented herein, respectively. Each of the apparatuses may comprise one or more processors and may be realized as a computing device.

### Brief Description of the Drawings

Further details, advantages and aspects of the present disclosure will become apparent from the following embodiments taken in conjunction with the drawings, wherein:
- Fig. 1A: schematically illustrates a spinal rod stabilizing multiple vertebrae;
- Fig. 1B: schematically illustrates pedicle screw placement using a guide wire;
- Fig. 1C: schematically illustrates pedicle screw placement planning with a selectable range for a screw transverse angle;
- Figs. 2A & B: schematically illustrate a surgical system with imaging capabilities;
- Fig. 2C: illustrates segmented patient image data;
- Fig. 2D: illustrates an exemplary screw suggestion via a GUI;
- Fig. 3: illustrates a flow diagram of a method for a user-specific selection of a model for determining an implant-related parameter;
- Fig. 4: schematically illustrates historical data for use in the selection process illustrated in Fig. 1; and
- Fig. 5: illustrates a flow diagram of a method for machine-training a set of different models.

### Detailed Description

The following description is related to spinal interventions. It will be apparent that the present disclosure can also be implemented in other surgical contexts. Moreover, while the following description focuses on bone screws as exemplary implants, it will be apparent that the implants may also have different configurations. For example, the implants may be realized as bone plates, bone nails or bone pins. Evidently, all these implants define implant-related parameters either inherently (e.g., in terms of an implant dimension such as length or diameter) or with respect to a patient anatomy (e.g., in terms of an orientation relative to a bone).

Spinal interventions have become a widespread surgical treatment and are currently performed either manually by a surgeon, automatically by a surgical robot, or semiautomatically by a surgeon using robotic assistance. To guarantee proper surgical results, spinal interventions require surgical planning and intra-operative imaging to verify that the ongoing surgical procedure conforms to the surgical plan.

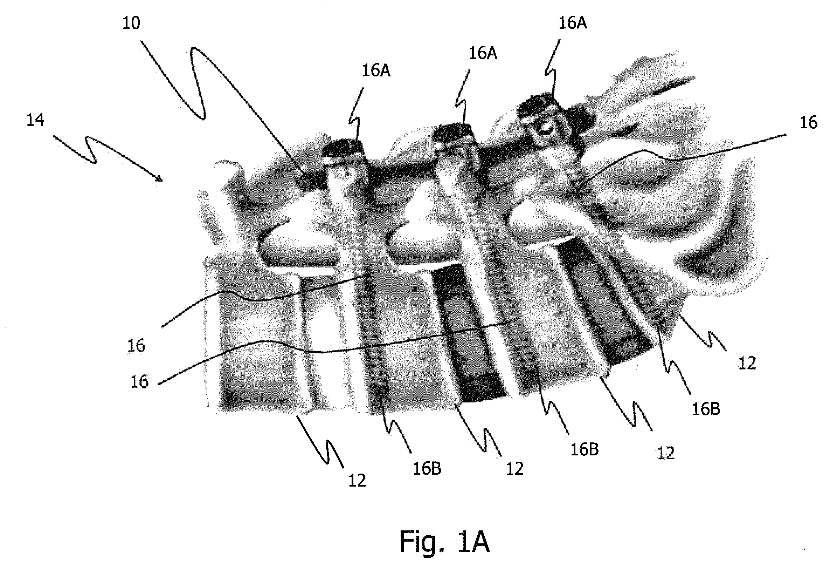

With reference to Fig. 1A, in some spinal interventions a pre-formed rod 10 is implanted to stabilize two or more vertebrae 12 of a patient's spine 14. The rod 10 may be pre-formed prior to its implantation according to a planned shape of the spine 14. After implantation, the rod 10 stabilizes the spine in accordance with the planned shape.

Fig. 1A shows that the pre-formed rod 10 is coupled to the vertebrae 12 using pedicle screws 16. Each pedicle screw 16 has a first end 16A, or head, attached to the rod 10 and an opposite second end 16B, or tip, anchored in a particular vertebra 12. The pedicle screws 16 enter the vertebrae 12 at a planned bone entry points and are oriented along planned screw extensions. The extension of a particular pedicle screw 16 in a particular vertebra 12 may be planned pre-operatively based on pre-operatively acquired image data of the spine 14. In other scenarios, pedicle screw placement is planned intra-operatively.

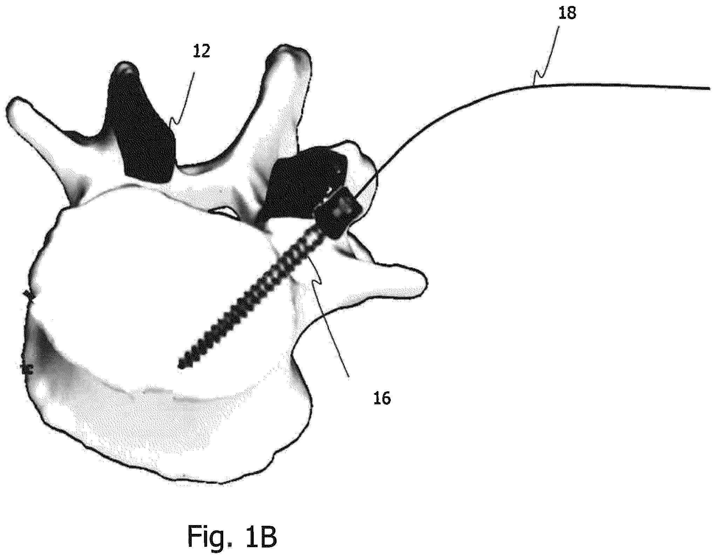

Pedicle screw placement can be facilitated using a guide wire 18, as illustrated in Fig. 1B. The guide wire 18 (such as a Kirschner wire, also called K wire) helps to guide a cannulated pedicle screws 16 to its desired bone entry point and along a desired trajectory. Surgical navigation techniques can be used to guide a user in inserting the guidewire 18 and the pedicle screws 16 (with or without guidewire 18) into bone.

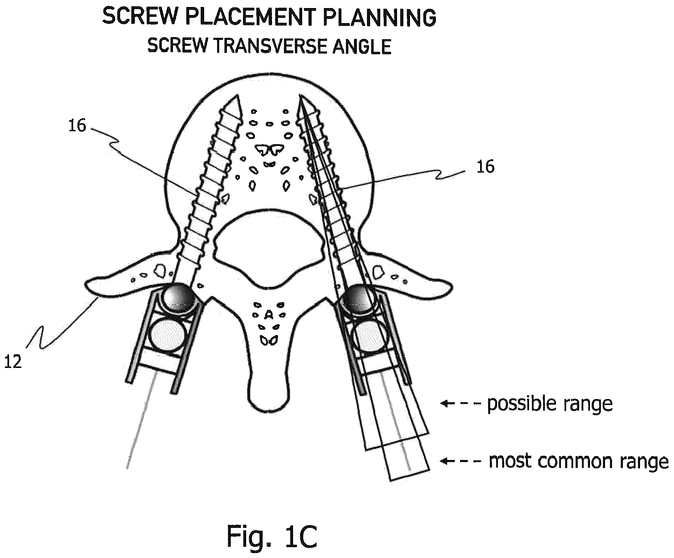

For the selection and placement of a dedicated pedicle screw 16 with respect to a dedicated vertebra 12 a user, e.g., a surgeon, normally has to decide between multiple options for each of multiple screw-related parameters like screw length and screw diameter as well as the extension of the dedicated pedicle screw 16 in the dedicated vertebra 12. During the planning of a placement of the pedicle screw 16, the user either manually selects one value for each parameter or manually adapts automatically suggested options. Fig. 1C schematically illustrates a planning visualization of a dedicated vertebra 12 with two dedicated pedicle screws 16 being placed therein. For the pedicle screw 16 on the right-hand side of the vertebra 12, a most common range and a possible range for a transverse angle of the pedicle screw 16 relative to the vertebra 12 are illustrated. The most common range defines a range of transversal angles which is selected by most users during planning of the placement of the pedicle screw 16. The possible range defines a range of transversal angles in which the transversal angle of the placed pedicle screw 16 may be selected in specific patient cases or in case a screw 16 with a smaller diameter is selected..

The selection of a dedicated transversal angle (as an exemplary implant-related parameter) is performed by the user during the planning phase based on his or her personal preference. The selected transversal angle, as one parameter determining the screw trajectory, will then form the basis for guiding placement of the guidewire 18 or the pedicle screw 16 (see Fig. 1C) using surgical navigation techniques. The personal preference of the user may be based on the medical field of the user (e.g., orthopedic surgery or neuro-surgery), the experience and skills of the user and auxiliary patient data like bone density, diagnosis and age of a patient, for example.

The selection of a dedicated transversal angle needs to be performed by the user for each pedicle screw 16, and there exist further screw-related parameters (such as pedicle screw diameter, or sagittal angle in respect to the vertebral endplate) that need to be selected, adapted or at least confirmed. Evidently, the manual selection or adaption of such parameters is time consuming and imposes a high cognitive load on the user. Moreover, less experienced users may specifically struggle upon being confronted with seldomly encountered patient anatomy constellations (e.g., in terms of a low bone density, or scoliosis cases where it is difficult to place screws inline for rod placement). As such, users will benefit from automated suggestions of screw-related parameters that are determined at least in part based on the personal preferences of a user. In this way, time and cognitive load required for a manual adaption are reduced. Evidently, the parameter suggestions should be of a high quality already before the user has expressed any or a significant number of personal preferences to enhance acceptance of the parameter suggestions.

For automatically suggesting implant-related parameters in the context of the placement of an implant (such as a pedicle screw 16), a patient anatomy in which the implant is to be placed (e.g., a spine 14 or one or more vertebrae 12) has to be identified. Such an identification may take place based on pre-operatively or intra-operatively acquired patient image data.

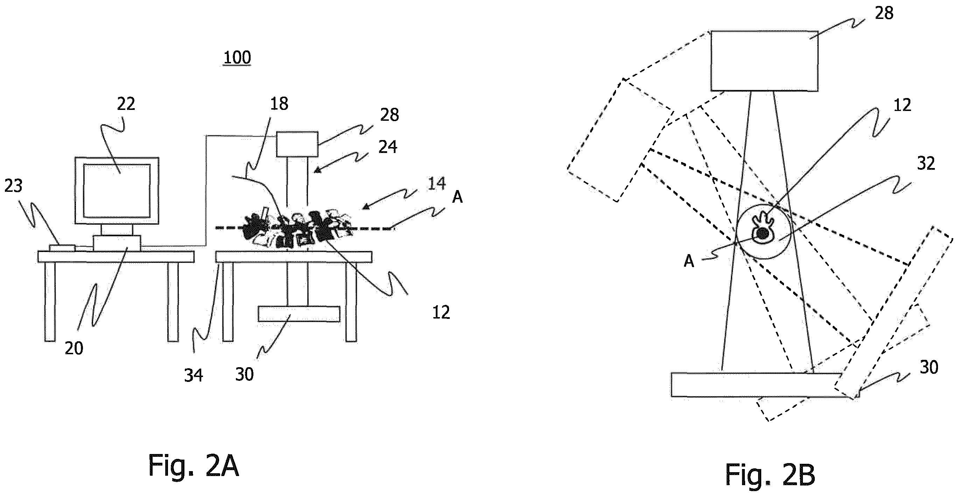

Fig. 2A schematically illustrates an exemplary surgical system 100 that is configured to acquire and optionally process patient image data indicative of a patient anatomy 12, 14 in which an implant 16 is to be placed. The system 100 is further configured to suggest implant-related parameters to a user. The system 100 may operate on the basis of patient image data for visualization, verification, navigation or other purposes.

The surgical system 100 illustrated in Fig. 2A comprises an apparatus 20 configured for receiving and processing patient image data and for suggesting implant-related parameters to a user. In some variants, the apparatus 20 may be part of, or include, a surgical navigation system. In Fig. 2A, the apparatus 20 is realized as a locally deployed computer system comprising one or more processors and memory storing program code for controlling operation of the one or more processors. Alternatively, the apparatus 20 may be realized in the form of a remote server or in the form of cloud computing resources. In other variants, the apparatus 20 may take the form of a tablet computer or other mobile device.

The system 100 of Fig. 2A further comprises an output device 22 configured to output information that has been derived based on the patient image data by the apparatus 20 (e.g., for information, verification, modification or confirmation purposes, or for navigation purposes). In particular, the output device 22 is configured to output suggestions for implant-related parameters to a user. In the scenario of Fig. 2A, the output device 22 is a display device configured to visually output the suggested implant-related parameters to the user, optionally in the context of (e.g., superimposed on) a visualization of the patient image data or information derived therefrom. In other variants, the output device 22 may be configured to (e.g., additionally or alternatively) output one or more of acoustic and haptic information. The output device 22 may be realized as, or comprise, a computer monitor, an augmented reality device (e.g., a head-mounted display), a loudspeaker, an actuator configured to generate haptically detectable information, or a combination thereof. The system 100 also comprises an input device 23 such as a keyboard or a mouse. The input device 23 enables the apparatus 20 to receive user feedback on the suggested implant-related parameters.

Operation of the apparatus 20 is based on input data. The input data comprise patient image data obtained on the basis of one or more surgical imaging techniques. For this reason, the surgical system 100 of Fig. 2A further comprises an imaging apparatus 24 configured to pre-operatively or intra-operatively acquire image data of one or more vertebrae 12 of the patient's spine 14. In the exemplary scenario illustrated in Fig. 2A, the imaging apparatus 24 is a C-arm with cone beam computer tomography (CBCT) imaging capabilities. In other scenarios, the imaging apparatus 24 is a conventional CT scanner that generates slice images, an EOS scanner or an X-ray apparatus. In still further scenarios, the imaging apparatus 24 utilizes a magnet resonance- (MR-) based imaging technique.

The exemplary CBCT-based imaging apparatus 24 of Fig. 2A comprises a radiation source 28 configured to generate a cone-shaped beam of radiation. Moreover, the imaging apparatus 24 has a flat-panel detector 30 configured to detect the radiation beam projected by the radiation source 28 through one or more of the vertebrae 12 with the one or more guide wires 18 being placed there. The detector 30 is configured to generate image data representative of two-dimensional projection images of the vertebrae 12. As illustrated in Fig. 2B, such projection images of an imaged volume 32 containing the vertebrae 12 are taken at two more angular orientations of the C-arm relative to an imaginary longitudinal axis A of the vertebrae 12. From the resulting two-dimensional images, in some variants three-dimensional image data are derived using reconstruction (e.g., back-projection) techniques. The reconstruction may be performed either by the imaging apparatus 24 itself or by the apparatus 20.

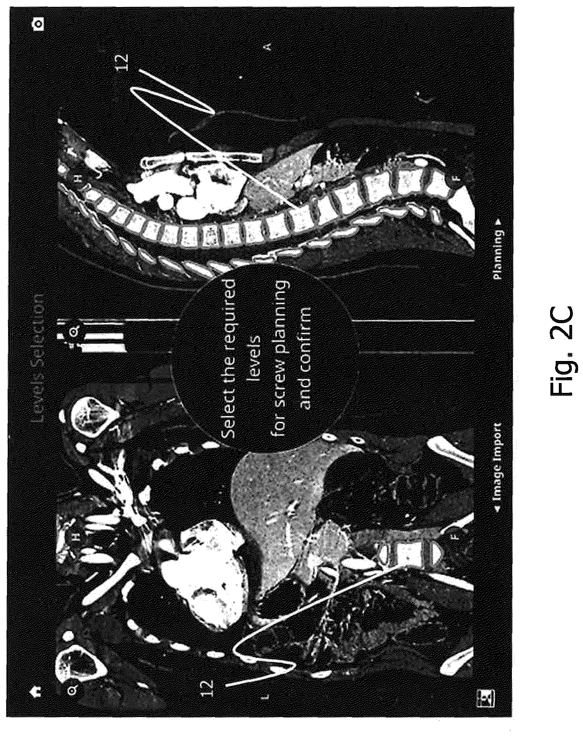

The imaging apparatus 24 or the apparatus 20 is configured to process the patient image data acquired by the imaging apparatus 24. Fig. 2C illustrates exemplary patient image data imported and processed by the imaging apparatus 24 or the apparatus 20 and visualized by the output device 22 in two perpendicular planes.

In the present realization, the apparatus 20 is configured to process the image data by applying an image segmentation algorithm so as to identify bone contours in the patient image data. Such a segmentation helps it to differentiate the vertebrae 12, i.e., to identify different vertebral levels, of the spine 14, as shown in Fig. 2C. The segmenting may be performed on a per-pixel or per-voxel basis of the underlying image data. In the case of CT image data, the segmenting may be based on an analysis of the Hounsfield values comprised by the CT or on a region growing algorithm utilizing a seed point. In some variants, the poses of the segmented vertebrae 12 are determined in a coordinate system of the patient image data.

Based on the visualization of the segmented vertebrae 12 as illustrated in Fig. 2C, a user may select via the input device 23 one or more vertebrae 12 for planning of a pedicle screw placement therein (e.g., by clicking on the vertebrae 12 to be treated). As an example, the user may select the sequence of vertebra levels L3 to L5 that are to be stabilized via a rod 10 as explained above with reference to Fig. 1A.

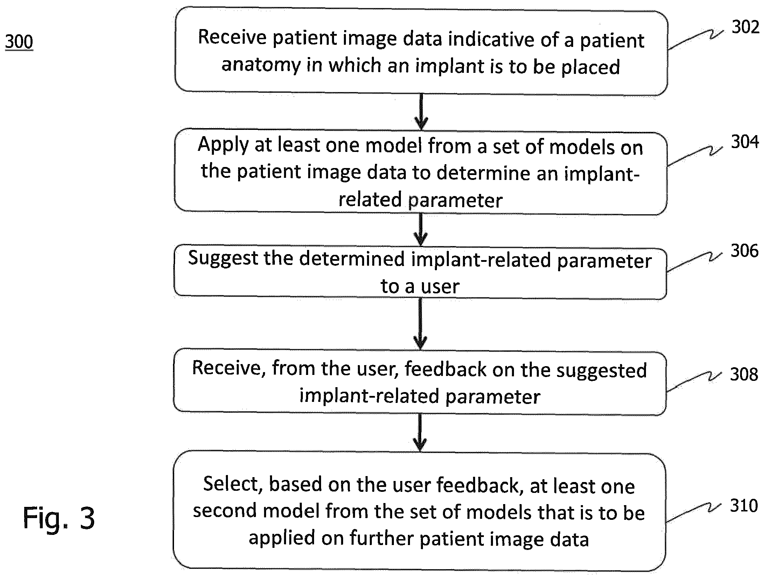

With regard to the planning of one or more implant-related parameters for the fixation screw placement (e.g., for placement of pedicle screws), the apparatus 20 is further configured apply artificial intelligence. In particular, the apparatus 20 is configured for a user-specific selection of a machine-trained model for determining such parameters, as will now be explained in greater detail with reference to the flow diagram 300 of Fig. 3. The procedure illustrated in Fig. 3 may start with a user logging into the apparatus 20 (or a computer system interfacing the apparatus 20) with a personalized account and creating or retrieving a specific patient case. The personalized account ensures that there exists a dedicated user context (e.g., in terms of dedicated historical data acquired for this user).

In step 302 of Fig. 3, the patient image data acquired by the imaging apparatus 24 are received by the apparatus 20 (e.g., retrieved from a database). The image data may be 3-dimensional data. The image data may be received in a DICOM format. Optionally, the received patient image data are then processed in step 302 (e.g., segmented as explained with reference to Fig. 2C) or have already been processed.

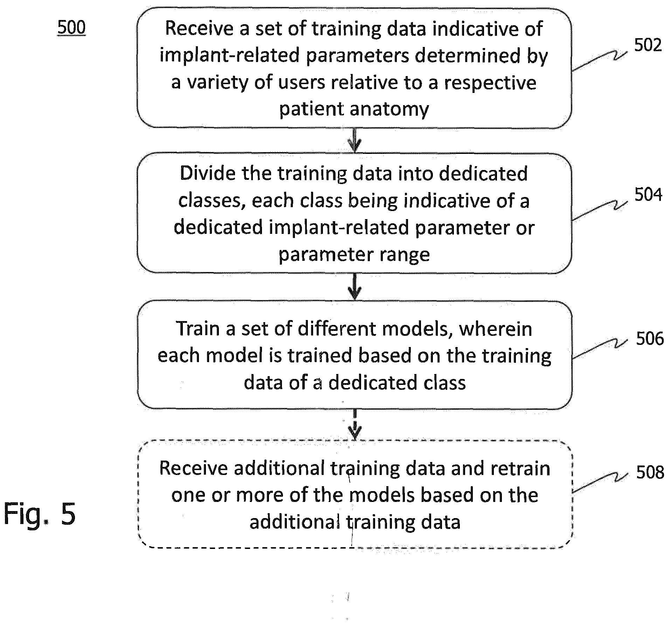

In step 304 of Fig. 3, at least one model out of a set of machine-trained models for determining an pedicle screw-related parameter is applied to the patient image data received in step 302. The machine-trained models can take any form, such as neural networks, models generated by linear regression, and so on. Generation of the machine-trained models will be explained in greater detail with reference to the flow diagram 500 of Fig. 5 below.

There exist various possibilities how to determine in step 302 the at least one machine-trained model that is to be applied to the patient image data. In some variants, the at least one machine-trained model is selected out of a set of models. For example, the at least one model may be selected at random out of the model set or may have been pre-configured. In still other variants, the at least one model is selected based on historical data generated during previous implant placement plannings performed by the dedicated user ("John Smith") who has triggered the procedure of Fig. 3.

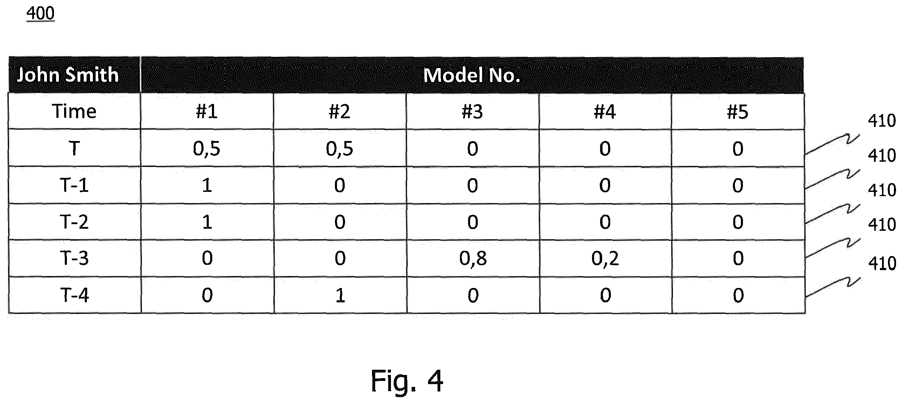

Fig. 4 illustrates an example of historical data 400 (in form of a table, but any other data structure could be used) that may be used in step 302. The historical data 400 is associated with the dedicated user ("John Smith") and comprises five data items 410 (table lines). Each data item 410 comprises an entry (table column) for each of five models. It will be appreciated that the number of models indicated in a particular data item 410 may vary. In particular, the number of models may be larger or smaller than five. In particular, each data item may only include information for a single model.

Each data item 410 is associated with a previous ("historic") implant placement planning performed by the user. In some variants, each data item 410 may be associated with the same selection of vertebrae 12 as previously input by the user (as explained above with reference to Fig. 2C) but for a different patient (optionally with a similar or different diagnosis, age and size). In such variants a dedicated set of models may be provided for each possible selection of vertebra 12, and the model selection procedure illustrated in Fig. 3 may be limited to the set of models that corresponds to the user's vertebrae selection. In other variants, the same set of models is used independent from the user's vertebrae selection.

Further, each data item 410 shown in Fig. 4 comprises a weighting factor for each of the five models (table columns). As a result, each data item 410 is indicative of a single model or of multiple, e.g., two, models to be used for generating one or more implant-related parameters when being applied to patient image data. Each data item 410 may be indicative of the best matching models as determined for a previous implant placement planning performed by the user. In some variants the historical data 400 may be directed at dedicated screws 16 in dedicated vertebra 12, i.e., a data item 410 may be associated with the placement planning of a single pedicle screw 16. In some variants the historical data 400 may be directed at a set of pedicle screws 16 for a dedicated selection of vertebrae 12, i.e., a data item 410 may be associated with the placement planning of multiple or all pedicle screws 16 for a selection of vertebrae 12 (as explained above with reference to Fig. 2C).

Taking into account the historical data 400, determining the at least one model to be applied to the patient image data in step 304 may result in determining the model most often indicated in the historical data items 410. In the example illustrated in Fig. 4, model #1 would thus be applied. In other cases, two or more models most often indicated in the historical data items 410 may be selected, and an interpolation (e.g., a linear combination) of the selected models may be applied. In the example illustrated in Fig. 4, a linear combination of models #1 and #2 may thus be applied (possibly based on a weighting that depends on the accumulated weightings of each model, here 2,5 : 1,5). In still further cases, the newest data item 410 (here for point in time T) is selected to define the model(s) to be applied in step 304 (here, a linear combination of models #1 and #2 with a weighting of 0,5 : 0.5). Of course, there exist various further possibilities for determining the model to be applied on the patient image data based on the historical data 400.

An interpolated model (e.g., a linear combination) of the selected models may be added to the set of models. For example, a hierarchical clustering method like single linkage clustering may be utilized to determine accumulations of specific interpolations and the most often used interpolated models are added to the set of models. In other variants, all models above a threshold accumulation may be added. In still other variants, an interpolated model will be exchanged for a model least often indicated in the historical data so that the number of models comprised by the set of models may be held constant.

In some variants, for example if there exist no historical data 400 for a specific user, the at least one model to be applied in step 304 may be randomly selected from a given set of models or based on the preference of a specific group of users to which the user belongs (e.g., neuro-surgeons or surgeons of a particular hospital).

In some variants, auxiliary information indicative of at least one of (i) a patient-related parameter such as sex and (ii) a user-related parameter such as membership of a user group is received by the apparatus 20 (e.g., is input by the user or automatically detected based on the patient image data received in step 302). In such a case, the (at least one) model to be applied in step 304 may also be determined based on the received auxiliary information. The auxiliary information may be reflected in the set of models from which in step 304 the model to be applied is determined (e.g., there may exist different model sets for male and female patients and/or for neuro-surgeons and orthopedic surgeons). Moreover, the historical data (e.g., each data item 410) may be indicative of the auxiliary information. For example, for a given user there may exist dedicated historical data for male patients and for female patients. In such a case, the (at least one) model to be applied in step 304 may be specifically selected based on the historical data 400 that correspond the to the currently applicable auxiliary information. If, for example, the patient to be treated is female, model selection will be based on historical data 400 gathered from female patients treated by John Smith.

Application of one or more models in step 304 to the patient image data received in step 302 will yield one or more implant-related parameters. As an example, the model set may comprise multiple neural networks, and the patient image data may be input to one or more of the neural networks that have been selected from the model set as explained above to yield a dedicated pedicle screw dimension (e.g., in terms of a screw diameter and a screw length). Additionally, or in the alternative, the implant-related parameter thus obtained may by indicative of a dedicated pedicle screw orientation (e.g., extension) relative to the patient's anatomy as indicated in the patient image data.

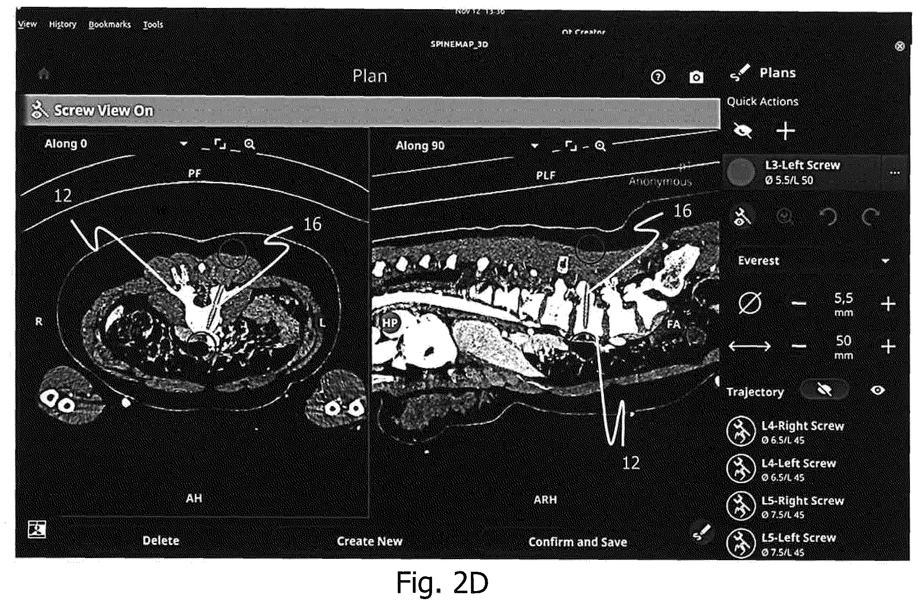

Returning to Fig. 3, the illustrated method then proceeds in step 306 to suggest to the user the implant-related parameters determined by applying the at least one selected model on the patient image data. The implant-related parameters may be suggested to the user via the output device 22 as explained with reference to Fig. 2A above. For example, the suggested screw diameter(s) and screw length(s) may be visualized on a graphical user interface (GUI) as shown in Fig 2D. The GUI enables the user to change individual suggested screw-related parameters. Screw diameter and length are shown on the right side panel, and the screw position can be changed with the circular handles shown in the sagittal and axial view. Additionally, or in the alternative, the (optionally segmented, see Fig. 2C) patient image data may be visualized and the suggested pedicle screw orientation (e.g., in terms of a screw transverse angle, see Fig. 1C) may be superimposed on the visualized patient image data.

In response to the suggesting the one or more implant-related parameters to the user in step 306, the user may either confirm the suggested parameters or manually adapt the parameters via the input device 23. In other words, the apparatus 20 may receive user feedback on the suggested implant-related parameter(s), see step 308.

For example, the user may change a suggested screw length or a suggested screw transverse angle. Alternatively, or in addition, the user may simply confirm the suggestion(s) of step 306. Using historical data 400 for model selection in step 304 may increase the probability for a user confirmation in step 308. In other words, using historical data 400 may reduce at least one of the need for manually adapting the suggested parameters and the magnitude by which the parameters have to be adapted.

In step 310, the method then continues with (again) selecting, based on the user feedback received in step 308, at least one model from the set of models that is to be applied on patient image data. These patient image data will typically, but not necessarily be different from the patient image data received in step 302. For example, steps 302 to 308 may be performed in relation to image data relating to a first patient at point in time T+x (see timing indicated in the historical data 400 of Fig. 4), and step 310 may be performed in relation to image data relating to a second patient at point in time T+x+y . Assuming that x = 1 and y =1, the historical data 400 available at point in time T+2 may include a further historical data item 410 for point in time T+1, and this further historical data item 410 may be indicative of the user feedback received in step 308. As such, steps 302 to 310 can belong to an iterative procedure, wherein step 310 belongs to a later iteration compared to steps 302 to 308. In other words, step 310 may be performed to comprise one or more of steps 302 to 308 (in particular step 304), but in a later iteration and possibly based on different patient image data and "updated" historical data that reflect the user feedback received in an earlier iteration. It is to be expected that the need for performing any manual adaptations in step 308 will generally decrease with each iteration as the volume of historical data 400 becomes larger and, possibly, converges in regard to the "best" model to be selected in view of the repeated user feedback. Moreover, since the models to be applied in step 304 may be re-trained based on the results of earlier iterations, the model quality will also increase, thus further decreasing the need for any manual adaptations in step 308.

The model selected in step 310 may simply be the model selected in step 304, for example if the user feedback received in step 308 comprises a confirmation of the suggested implant-related parameter. In case that the user feedback received in step 308 comprises a manual adaptation, several (e.g., each) of the models of the model set may be applied in step 310 to the patient image data received in step 302. As a result, a corresponding set of implant-related parameters will be obtained (i.e., application of each model will result in one or more model-specific implant-related parameters). The one or more implant-related parameters generated by applying a model are then for each model compared to the manually adapted one or more parameters received in step 308. The model whose application results in one or more implant-related parameters with the least deviation to the manually adapted one or more parameters may be identified.

In other words, each "member" of the set of implant-related parameters obtained by applying several (e.g., each) of the models of the model set may then be fitted to the manually adapted implant-related parameter(s) resulting from step 308 to obtain a corresponding fit. Each fit may, for example, be indicative of a deviation of the corresponding model "output" from the user-adapted parameter. Dependent on the resulting set of fits, at least one model may be identified from the set of models. For example, the model that resulted in the best fit may be identified. In some variants, multiple models may be identified (e.g., the two models that fit best). The identified models may then be associated with weighting factors (e.g., depending on their corresponding fit) for an interpolation as explained hereinabove.

The method may then proceed with generating a new historical data item 410 for the user and indicative of the at least one model identified as explained above. The new historical data item 410 may only include the model with the best fit (such as model #1 in the data item 410 for point in time T of Fig. 4) or two or more models with associated weighting factors (such as models #1 and #2 in the data item for point in time T-1 of Fig. 4).

As a result of the fitting procedure, the new historical data item 410 will be indicative of the user feedback received in step 308. By generating new historical data items 410, the model selection as explained with reference to step 304 iteratively adapts to the preferences of the user. In general, the more historical data items 410 and/or the more recent historical data items 410 are taken into account, the closer the implant-related parameter suggested in step 306 will be to the preference of the user. In some variants, the maximum number of historical data items 410 taken into account may be limited so that a change in the preferences of the user may be reflected faster in future suggestions.

By utilizing the iteratively refined historical data 400 for adapting the suggestions in step 304 to the preferences of the user, the quality of the suggestions (from the perspective of a particular user) can be gradually improved without necessarily re-training the machine-trained models. As such, a high base quality of the machine-trained models can be maintained since the risk of lowering the quality by re-training the models, e.g., based on user feedback from inexperienced users, can be eliminated.

Steps 302 to 308 and 310 may be performed in dedicated planning procedures for implant placements. Those planning procedures may be performed pre-operatively or inter-operatively. The results of the planning procedures may then be used for surgical navigation purposes during surgical interventions.

In the following, an exemplary procedure for obtaining the machine-trained models used in the procedure of Fig. 3 will be described with reference to the flow diagram 500 of Fig. 5. In some realizations, the apparatus 20 may be configured for machine-training a set of models, but the machine training can also be performed by another apparatus (e.g., in a cloud computing context) and, in particular, in an off-line mode. For example, the procedure illustrated in Fig. 5 may be performed by a central processing component that has received a large volume of training data from multiple distributed apparatuses 20.

The machine-trained models thus obtained may take the form of neural networks (e.g., a deep neural network or a convoluted neural network or another suitable neural network as known in the art), models obtained by linear regression, and so on.

Now referring to Fig. 5, in step 502 a set of training data is received (e.g., from a database). The training data is indicative of implant-related parameters determined by a variety of users relative to a respective patient anatomy, such as one or more vertebrae 12 of a patient's spine 14 (see Fig. 1A). For example, the training data may be indicative of implant-related parameters (e.g., pedicle screw selections and placements) of multiple different experienced users. The training data may be indicative of the implant-related parameters relative to (e.g., a segmented contour of) the patient anatomy. The training data may thus include patient image data. In some cases, the training data may be obtained using an imaging technique as discussed above with reference to Figs. 2A and 2B but after the implants have been placed (so as to be indicative of the implant-related parameters relative to the patient anatomy). Additionally or in the alternative, the training data may include numerical values of dimensions of the implants that have been placed. In such or other cases, the training data may include planning data indicative of the implant-related parameters.

The training data may comprise, for the example of pedicle screws 16, data indicative of (i) delta parameters, such as the a screw diameter change per vertebra level, an angle of a screw orientation relative a vertebra, (ii) anonymized metrics, e.g. breaching surface (overlap of screw surface with vertebra segmentation manifold), bone density mean within the volume the planned screw 16 will occupy, (iii) reduced imaging data volume, e.g., cut outs in the region of the planned screw 16, and (iv) full PHI image data with placed screws 16.

In some variants, different sets of models may be trained for different anatomies, e.g., one set for each sequence of vertebrae that are to be stabilized. The training data may also comprise auxiliary information as explained above (e.g., indicative of at least one of a respective patient diagnosis, patient age, and/or a surgical division of the respective user). In some variants, different sets of models may thus be trained for different surgical divisions, patients of different sexes, and so on.

In step 504, the training data received in step 502 are divided into dedicated classes. Each class is indicative of a dedicated implant-related parameter or parameter range. For example, a first parameter range is indicative of a first portion of the most common range for the transversal angle shown in Fig. 1C, and a second parameter range is indicative of a second portion of the most common range for the transversal angle different from the first part. The different classes may also reflect different implant dimensions (e.g., pedicle screw lengths) and so on. As such, users with different preferences may be associated with different classes.

In some variants, the apparatus 20 is configured to automatically divide the training data, e.g., define the parameter ranges, based on a similarity metric for one or more implant-related parameters. Therefore, the apparatus 20 is enabled to perform a cluster analysis of the training data. For example, the apparatus 20 may be configured to map vertebral levels indicated in the training data to a reference spine and to perform a cluster analysis on screw positions, e.g., with a single linkage hierarchical clustering algorithm. The similarity metric may define, e.g., a weighted sum of the differences between different implant-related parameters, e.g., a screw head 16A and screw tip 16B position relative to the patient anatomy, a screw diameter of a pedicle screw 16, and so on.

In step 506, a set of models is trained based on the dedicated classes. In particular, each model of the set of models is trained based on one dedicated class of training data. As a result, each model is associated with one of the classes and differs from the other trained models.

In an optional step 508, the method may proceed with receiving additional training data and re-training one or more of the models based on the additional training data. The additional training data may be based on the implant-related parameters confirmed or adapted in step 308 of Fig. 3. In some variants, the received additional training data may be reviewed, e.g., by experienced users prior to re-training one or more of the models to ensure high quality of the additional training data.

As has become apparent from the above description of exemplary realizations of the present disclosure, the model selection and model training techniques presented herein reduce the cognitive load on the user and lead to a more efficient surgical planning procedure. The result of the surgical planning procedure may then enable a more efficient surgical navigation during the actual surgical intervention. The techniques presented herein can be based on expert-level training data while at the same time iteratively considering user feedback on preferences of potentially less experienced users. As such, the need for manual adaptations of automatically suggested implant-related parameters will decrease over time, and the surgical results can be improved.

## Claims

1. A computer-implemented method for a user-specific selection of a model for determining an implant-related parameter, wherein a set of different machine-trained models is provided, with each model being indicative of a dedicated implant-related parameter for a dedicated patient anatomy (12, 14) the method comprising:
receiving (302) first patient image data indicative of a patient anatomy (12, 14) in which an implant (16) is to be placed;
applying (304) at least one first model from the set of models on the first patient image data to determine an implant-related parameter;
suggesting (306) the determined implant-related parameter to a dedicated user;
receiving (308), from the user, feedback on the suggested implant-related parameter, the user feedback comprising one of a confirmation of the suggested implant-related parameter and an adaptation thereof; and
selecting (310), based on the user feedback, at least one second model from the set of models that is to be applied on second patient image data.

2. The method of claim 1, comprising at least one of:
selecting the at least one first model based on at least one first historical data item (410) associated with the user and indicative of at least one third model of the set of models; and
generating at least one second historical data item (410) associated with the user and indicative of the at least one second model.

3. The method of claim 2, wherein there exist multiple first historical data items (410), and wherein selecting the at least one first model comprises at least one of:
i) determining the model most often indicated in the multiple first historical data items (410); and
ii) an interpolation based on at least some of the models indicated in the multiple first historical data items (410).

4. The method of claim 2 or 3, wherein the user feedback comprises an adaptation of the suggested implant-related parameter, and wherein the step of generating the at least one second historical data item (410) comprises:
utilizing each of the models from the model set to determine, based on the first patient image data, a respective implant-related parameter;
determining, for each respective implant-related parameter, a respective fit to the adapted implant-related parameter; and
generating the at least one second historical data item (410) based on one or more of the respective fits.

5. The method of any preceding claim, further comprising;
receiving auxiliary information indicative of at least one of a patient-related parameter and a user-related parameter, and wherein
at least one of the first and the second model is also determined based on the received auxiliary information.

6. The method of claim 5, wherein
the auxiliary information is reflected in the set of models.

7. The method of claim 5 or 6, when depending on at least claim 2, wherein
each historical data item (410) is indicative of the auxiliary information, and
the at least one first model is selected based on the at least one first historical data item (410) that is associated with the user and indicative of the at least one third model of the set of models and of the auxiliary information.

8. The method of any preceding claim, wherein
the implant (16) is a bone screw (16) and the dedicated implant-related parameter is indicative of at least one of a dedicated orientation of the screw relative to the patient's anatomy (12, 14), a dedicated position of a head (16A) of the screw (16) relative to the patient's anatomy (12, 14), a dedicated position of a tip (16B) of the screw (16) relative to the patient's anatomy (12, 14), a dedicated screw length and a dedicated screw diameter.

9. The method of any preceding claim, wherein the patient anatomy (12, 14) is a spine (14), wherein there exists a dedicated set of models for each of multiple sets of one or more vertebrae (12) of the spine (14) that are to be treated, and further comprising:
receiving a user input on a set of one or more vertebrae (12) to be treated;
and wherein model selection is also based on the user input.

10. The method of any preceding claim, wherein
suggesting the determined implant-related parameter to the user comprises visualizing the implant-related parameter relative to the first patient image data.

11. A computer-implemented method for machine-training a set of different models, each model being configured for determining a dedicated implant-related parameter with respect to a dedicated patient anatomy (12, 14), the method comprising:
receiving (502) a set of training data indicative of implant-related parameters determined by a variety of users relative to a respective patient anatomy (12, 14);
dividing (504) the training data into dedicated classes, each class being indicative of a dedicated implant-related parameter or parameter range; and
machine-training (506) a set of different models, wherein each model is trained based on the training data of a dedicated class.

12. The method of claim 11, wherein
dividing the training data is done responsive to a manual input.

13. The method of claim 11 or 12, wherein
dividing the training data is performed at least partially automatically based on a similarity metric for the implant-related parameter.

14. The method of claim 13, wherein
dividing the training data based on the similarity metric comprises performing a cluster analysis of the training data.

15. The method of any of claims 11 to 14, further comprising:
receiving additional training data indicative of one or more implant-related parameters that have been adapted in accordance with claim 1; and
retraining (508) one or more of the models based on the additional training data.

16. The method of any of claims 11 to 15, wherein
the training data are indicative of the implant-related parameters relative to a segmented contour of the patient anatomy (12, 14).

17. A computer program product comprising program code portions configured to cause a processor to carry out a method of any of the preceding claims when the program code portions are executed on the processor.

18. An apparatus (20) for a user-specific selection of a model for determining an implant-related parameter, wherein a set of different machine-trained models is provided, with each model being indicative of a dedicated implant-related parameter for a dedicated patient anatomy (12, 14), the apparatus (20) being configured to:
receive (302) first patient image data indicative of a patient anatomy (12, 14) in which an implant (16) is to be placed;
apply (304) at least one first model from the set of models on the first patient image data to determine an implant-related parameter;
suggest (306) the determined implant-related parameter to a dedicated user;
receive (308), from the user, feedback on the suggested implant-related parameter, the user feedback comprising one of a confirmation of the suggested implant-related parameter and an adaptation thereof; and
select (310), based on the user feedback, at least one second model from the set of models that is to be applied on second patient image data.

19. An apparatus (20) for machine-training a set of different models, each model being configured for determining a dedicated implant-related parameter with respect to a dedicated patient anatomy (12, 14), the apparatus (20) being configured to:
receive (502) a set of training data indicative of implant-related parameters determined by a variety of users relative to a respective patient anatomy (12, 14);
divide (504) the training data into dedicated classes, each class being indicative of a dedicated implant-related parameter or parameter range; and
train (506) a set of different models, wherein each model is trained based on the training data of a dedicated class.

20. The apparatus (20) of claim 18 or 19, configured to carry out the method of any of claims 2 to 10 or 12 to 16, respectively.

## Amended claims

### Amended claims in accordance with Rule 137(2) EPC.

1. A computer-implemented method for a user-specific selection of a model for determining an implant-related parameter, wherein a set of different machine-trained models is provided, with each model being indicative of a dedicated implant-related parameter for a dedicated patient anatomy (12, 14) and trained based on one dedicated class of training data, with each class being indicative of the dedicated implant-related parameter or a dedicated parameter range the method comprising:
receiving (302) first patient image data indicative of a patient anatomy (12, 14) in which an implant (16) is to be placed;
applying (304) at least one first model from the set of models on the first patient image data to determine an implant-related parameter;
suggesting (306) the determined implant-related parameter to a dedicated user;
receiving (308), from the user, feedback on the suggested implant-related parameter, the user feedback comprising one of a confirmation of the suggested implant-related parameter and an adaptation thereof; and
selecting (310), based on the user feedback, at least one second model from the set of models that is to be applied on second patient image data, wherein, if the received user feedback comprises a confirmation of the suggested implant-related parameter, the at least one second model is the first model.

2. The method of claim 1, comprising at least one of:
selecting the at least one first model based on at least one first historical data item (410) associated with the user and indicative of at least one third model of the set of models; and
generating at least one second historical data item (410) associated with the user and indicative of the at least one second model.

3. The method of claim 2, wherein there exist multiple first historical data items (410), and wherein selecting the at least one first model comprises at least one of:
i) determining the model most often indicated in the multiple first historical data items (410); and
ii) an interpolation based on at least some of the models indicated in the multiple first historical data items (410).

4. The method of claim 2 or 3, wherein the user feedback comprises an adaptation of the suggested implant-related parameter, and wherein the step of generating the at least one second historical data item (410) comprises:
utilizing each of the models from the model set to determine, based on the first patient image data, a respective implant-related parameter;
determining, for each respective implant-related parameter, a respective fit to the adapted implant-related parameter; and
generating the at least one second historical data item (410) based on one or more of the respective fits.

5. The method of any preceding claim, further comprising;
receiving auxiliary information indicative of at least one of a patient-related parameter and a user-related parameter, and wherein
at least one of the first and the second model is also determined based on the received auxiliary information.

6. The method of claim 5, wherein
the auxiliary information is reflected in the set of models.

7. The method of claim 5 or 6, when depending on at least claim 2, wherein
each historical data item (410) is indicative of the auxiliary information, and the at least one first model is selected based on the at least one first historical data item (410) that is associated with the user and indicative of the at least one third model of the set of models and of the auxiliary information.

8. The method of any preceding claim, wherein
the implant (16) is a bone screw (16) and the dedicated implant-related parameter is indicative of at least one of a dedicated orientation of the screw relative to the patient's anatomy (12, 14), a dedicated position of a head (16A) of the screw (16) relative to the patient's anatomy (12, 14), a dedicated position of a tip (16B) of the screw (16) relative to the patient's anatomy (12, 14), a dedicated screw length and a dedicated screw diameter.

9. The method of any preceding claim, wherein the patient anatomy (12, 14) is a spine (14), wherein there exists a dedicated set of models for each of multiple sets of one or more vertebrae (12) of the spine (14) that are to be treated, and further comprising:
receiving a user input on a set of one or more vertebrae (12) to be treated;
and wherein model selection is also based on the user input.

10. The method of any preceding claim, wherein
suggesting the determined implant-related parameter to the user comprises visualizing the implant-related parameter relative to the first patient image data.

11. A computer-implemented method for machine-training a set of different models, each model being configured for determining a dedicated implant-related parameter with respect to a dedicated patient anatomy (12, 14), the method comprising:
receiving (502) a set of training data indicative of implant-related parameters determined by a variety of users relative to a respective patient anatomy (12, 14);
dividing (504) the training data into dedicated classes, each class being indicative of a dedicated implant-related parameter or parameter range;
machine-training (506) a set of different models, wherein each model is trained based on the training data of a dedicated class; and
determining a user-specific selection of a model for determining an implant-related parameter based on the machine-trained set of different models.

12. The method of claim 11, wherein
dividing the training data is done responsive to a manual input.

13. The method of claim 11 or 12, wherein
dividing the training data is performed at least partially automatically based on a similarity metric for the implant-related parameter.

14. The method of claim 13, wherein
dividing the training data based on the similarity metric comprises performing a cluster analysis of the training data.

15. The method of any of claims 11 to 14, further comprising:
receiving additional training data indicative of one or more implant-related parameters that have been adapted in accordance with claim 1; and
retraining (508) one or more of the models based on the additional training data.

16. The method of any of claims 11 to 15, wherein
the training data are indicative of the implant-related parameters relative to a segmented contour of the patient anatomy (12, 14).

17. A computer program product comprising program code portions configured to cause a processor to carry out a method of any of the preceding claims when the program code portions are executed on the processor.

18. An apparatus (20) for a user-specific selection of a model for determining an implant-related parameter, wherein a set of different machine-trained models is provided, with each model being indicative of a dedicated implant-related parameter for a dedicated patient anatomy (12, 14) and trained based on one dedicated class of training data, with each class being indicative of the dedicated implant-related parameter or a dedicated parameter range, the apparatus (20) being configured to:
receive (302) first patient image data indicative of a patient anatomy (12, 14) in which an implant (16) is to be placed;
apply (304) at least one first model from the set of models on the first patient image data to determine an implant-related parameter;
suggest (306) the determined implant-related parameter to a dedicated user;
receive (308), from the user, feedback on the suggested implant-related parameter, the user feedback comprising one of a confirmation of the suggested implant-related parameter and an adaptation thereof; and
select (310), based on the user feedback, at least one second model from the set of models that is to be applied on second patient image data, wherein, if the received user feedback comprises a confirmation of the suggested implant-related parameter, the at least one second model is the first model.

19. An apparatus (20) for machine-training a set of different models, each model being configured for determining a dedicated implant-related parameter with respect to a dedicated patient anatomy (12, 14), the apparatus (20) being configured to:
receive (502) a set of training data indicative of implant-related parameters determined by a variety of users relative to a respective patient anatomy (12, 14);
divide (504) the training data into dedicated classes, each class being indicative of a dedicated implant-related parameter or parameter range; and
train (506) a set of different models, wherein each model is trained based on the training data of a dedicated class
determine a user-specific selection of a model for determining an implant-related parameter based on the machine-trained set of different models.

20. The apparatus (20) of claim 18 or 19, configured to carry out the method of any of claims 2 to 10 or 12 to 16, respectively.
